(19) **Europäisches Patentamt / European Patent Office / Office européen des brevets**

(11) **EP 1 964 855 B1**

(12) **EUROPEAN PATENT SPECIFICATION**

(45) Date of publication and mention of the grant of the patent:
**04.04.2018 Bulletin 2018/14**

(21) Application number: **06834798.8**

(22) Date of filing: **15.12.2006**

(51) Int Cl.:
*C08B 37/00* (2006.01)     *A23K 10/00* (2016.01)
*A23L 33/00* (2016.01)     *A61K 31/718* (2006.01)
*A61K 47/36* (2006.01)     *A61P 3/00* (2006.01)

(86) International application number:
**PCT/JP2006/325055**

(87) International publication number:
**WO 2007/072756 (28.06.2007 Gazette 2007/26)**

(54) **MINERAL ABSORPTION ENHANCER, FOOD AND FEEDING STUFF**

MINERALIENRESORPTIONSVERBESSERER, LEBENSMITTEL UND FUTTERMITTEL

AGENT FAVORISANT L'ABSORPTION DES MINERAUX, ALIMENT ET SUBSTANCE ALIMENTAIRE

(84) Designated Contracting States:
**BE CH DE FR GB LI NL**

(30) Priority: **19.12.2005 JP 2005365200**
**04.09.2006 JP 2006239092**

(43) Date of publication of application:
**03.09.2008 Bulletin 2008/36**

(73) Proprietor: **MATSUTANI CHEMICAL INDUSTRY CO., LTD.**
**Itami-shi, Hyogo 664-8508 (JP)**

(72) Inventors:
• **ICHIHARA, Takashi**
 **Kobe-shi, Hyogo 658-0022 (JP)**
• **MIYAZATO, Shoko**
 **Hyogo 664-0899 (JP)**
• **TAGAMI, Hiroyuki**
 **Hyogo 664-0842 (JP)**
• **KISHIMOTO, Yuka**
 **Hyogo 669-1537 (JP)**

• **HARA, Hiroshi**
 **Sapporo-shi, Hokkaido 060-8589 (JP)**

(74) Representative: **Hart-Davis, Jason et al**
 **Cabinet Beau de Loménie**
 **158, rue de l'Université**
 **75340 Paris Cedex 07 (FR)**

(56) References cited:
EP-A1- 1 475 390     JP-A- 5 176 719
JP-A- 8 070 842      JP-A- 11 255 803
JP-A- 2002 145 893   JP-A- 2004 307 768
JP-A- 2004 524 366   JP-A- 2005 532 294

• VERMOREL M. ET AL.: 'Energy value of a low-digestible carbohydrate, NUTRIOSE FB, and its impact on magnesium, calcium and zinc apparent absorption and retention in healthy young men' EUR. J. NUTR. vol. 43, no. 6, 2004, pages 344 - 352, XP003014286

**Description**

Technical Field

The present invention relates to a mineral-absorption promoter characterized in that it comprises an alkali metal salt of a citric acid bound hardly digestible reduced dextrin or a derivative thereof, as well as a food and a feed containing the same.

Background Art

In the recent gluttony age, calcium, magnesium, iron and zinc have generally been known as essential minerals, the intake of which is insufficient or prone to be deficient. Among them, the nutritionally required daily intake of calcium for an adult is set at a level on the order of 600 to 700 mg for the Japanese, but the intake thereof on the average does not reach its nutrient intake under the present conditions. It has been known that calcium binds to inorganic phosphoric acid at an alkaline pH to thus form an insoluble calcium phosphate and it is believed that the rate of calcium-absorption through the intestinal tracts is considerably low. For this reason, there has extensively been tried to develop a method for improving the ability of calcium to be absorbed through the intestinal tracts, while making the most use of any substance capable of binding to calcium, which prevents calcium from insolubilization under the environmental conditions within the intestinal tracts.

For instance, there has been known that the casein phosphopeptide (CPP) isolated from milk can promote the calcium-absorption (see Patent Documents 1 and 2 given below). However, the content thereof in the milk is very low, it is accordingly quite expensive and it is quite difficult to remove bitter peptides therefrom. Patent Document 3 discloses the calcium-solubilization effect of calcium citrate/calcium malate complex, but the complex has an acid taste and the use thereof in food is accordingly limited. Patent Document 4 discloses that phosphorylated sugar, in which at least two phosphoric acid residues are bonded to a glucan comprising 2 to 8 glucose molecules linked together through $\alpha$-1,4 bonds, can prevent any insolubilization of minerals inclusive of calcium. This phosphorylated sugar is produced by acting a plurality of hydrolases or glucosyltransferases on phosphoric acid moiety-containing starch, but this production method suffers from such a problem that it requires the use of quite complicated operations. Moreover, the phosphorylated sugar has such an apprehension that the stability thereof in the intestinal tracts is insufficient. Patent Document 5 discloses phosphorylated polysaccharides capable of promoting the absorption of calcium and a method for the preparation thereof. Each of these phosphorylated polysaccharides is a high molecular weight phosphorylated sugar obtained by acting inorganic phosphoric acid on a naturally occurring or synthetic polysaccharide, but the use thereof in a food is highly limited because of its high viscosity.

As materials for food other than those described above, which can promote the absorption of minerals, there have been proposed, for instance, hardly digestible oligosaccharides such as fructo-oligosaccharides and xylo-oligosaccharides; and dietary fibers such as decomposed guar gum products (see, for instance, Patent Document 6 and Non-Patent Document 1 specified below).

These dietary fibers have been known to promote the absorption of minerals. It has been believed that the mechanism of this would be as follows: the dietary fibers reach the large intestine without being digested under the action of the digestive enzymes at the digestive organ, they are then hydrolyzed and fermented by the action of enteric bacteria, short chain fatty acids such as acetic acid, propionic acid and butyric acid are accordingly generated to thus lower the pH value in the intestine and the solubility of minerals can thus be improved. In this connection, such solubilization of minerals due to the lowered pH value is an essential requirement for the promotion of mineral-absorption, but it is not a sufficient requirement. Moreover, there has likewise been known a mechanism of promoting the mineral-absorption independent of the fermentation in the large intestine and more specifically, a variety of factors would be involved in the absorption of minerals (see Non-Patent Document 1 specified later).

On the other hand, there have been known hardly digestible dextrins as water-soluble dietary fibers derived from starch other than the hardly digestible oligosaccharides (see, for instance, Patent Document 8). The hardly digestible dextrins are in common with the foregoing hardly digestible oligosaccharides and the decomposed guar gum products in such a point that they reach the large intestine without being digested under the action of the digestive enzymes at the digestive organ, they are then hydrolyzed and fermented by the action of enteric bacteria, but the energy value of the former is equal to 1 kcal/g, while that observed for the foregoing hardly digestible oligosaccharides or the decomposed guar gum products is about 1/2 time the foregoing energy value (see Non-Patent Document 2 specified later). These energy values are determined by the degree of fermentation in the large intestine and accordingly, the foregoing energy values clearly indicate that the hardly digestible dextrins can generate rather small amounts of short chain fatty acids through the fermentation in the large intestine. In fact, in the in vitro fermentation tests carried out using the microbial flora originated from the human feces, the hardly digestible dextrins can generate a small amount of overall organic acids through the fermentation for 24 hours as compared with the amount thereof generated through the fermentation of the fructo-ohgosaccharide hydrolyzates or decomposed guar gum products and decrease in the pH value is accordingly slight (see Non-Patent Document 3 specified later).

Patent Document 9 discloses an enteral nutrient composition containing branched maltodextrins and it states that the branched maltodextrins promote the mineral-absorption. However, Patent Document 10 discloses that the energy value of the branched maltodextrins amounts to 2 kcal, which corresponds to two times that observed for the hardly digestible dextrins.

Thus, it is not easy to predict that the hardly digestible dextrins can promote the mineral-absorption while taking into consideration the foregoing knowledge.

MATSUDA et al. reported that the glucose polymer (hardly digestible dextrin) derived from starch and citric acid are heated under dry conditions to thus form a glucose polymer carrying bound citric acid moieties thereto and has an ability of exchanging ions and that the resulting glucose polymer forms a water-soluble salt with calcium. However, they never referred to the ability thereof to absorb minerals (see Patent Document 11 specified below).

Patent Document 1: JP-A-03-240470;
Patent Document 2: JP-A-05-284939;
Patent Document 3: JP-A-56-097248;
Patent Document 4: JP-A-08-104696;
Patent Document 5: JP-A-2000-157186;
Patent Document 6: JP-A-07-252156;
Patent Document 7: JP-A-07-067575;
Patent Document 8: JP-B-04-043624;
Patent Document 9: JP-A-2004-5243661
Patent Document 10: JP-A-2004-524849;
Patent Document 11: JP-A-2004-307768;
Non-Patent Document 1: New Food Industry, 2001, Vol. 43, No. 12, pp. 35-44;
Non-Patent Document 2: Bulletin of the Dietary Fiber Society in Japan, 2005, Vol. 9, No. 1, pp. 34-46;
Non-Patent Document 3: Journal of Nutrition, 2000, 130(5): 1267-1273.

Disclosure of the Invention

Problems That the Invention is to Solve

It is an object of the present invention to provide a mineral-absorption promoter which can easily be applied to food, is resistant to digestive enzymes secreted by mammals, and has a function of promoting mineral-absorption through the intestinal tracts.

It is a further object of the present invention to provide food or feed comprising the foregoing mineral-absorption promoter.

**Means for the Soution of the Problems**

The inventors of this invention have found that hardly digestible dextrins can promote the mineral-absorption through experiments using animals although they are only weakly fermented by the enteric bacteria. The inventors have likewise found that the glucose polymer which has an ion-exchange function and is disclosed in the foregoing Patent Document 11 can promote mineral-absorption in rats as well. The present invention is defined in the claims and has been developed on the basis of these findings.

**Effects of the Invention**

The mineral-absorption promoter and the food and feed containing the same according to the present invention have an excellent function of promoting mineral- absorption and accordingly, they are effective for solving the problem concerning the insufficiency of routine or chronic mineral-intake. In addition, the mineral-absorption promoter of the present invention has a low energy value and accordingly, it would contribute to the efficient mineral-intake of modern people.

**Best Mode for Carrying Out the Invention**

In respect of the mineral-absorption promoter of the present invention, the minerals are nutrients essential for animals and specific examples thereof include at least one member selected from the group consisting of calcium, magnesium, iron and zinc.

In this specification, "hardly digestible dextrin" used as an effective component of the mineral-absorption promoter of the present invention means dextrins indigestible with any digestive enzyme, which can be prepared by fractionating the dextrin obtained through roasting or heating starch in the presence of an acid or fractionating the dextrin after acting, on the dextrin, an acid or a saccharide-hydrolyzing enzyme such as $\alpha$-amylase and glucoamylase, or a hydrogenated product thereof. Such hardly digestible dextrins are commercially available from, for instance, Matsutani Chemical Industry Co., Ltd under the trade names of FIBERSOL 2 and FIBERSOL 2H (hydrogenated product).

The derivatives of hardly digestible dextrins used as the effective component of the mineral-absorption promoter of the present invention have an effect of promoting mineral-absorption. Citric acid-bound hardly digestible dextrins in which citric acid moieties are bonded to hardly digestible dextrins include citric acid-bound glucose polymers (see Patent Document 11) represented by citric acid-bound hardly digestible dextrins wherein citric acid moieties are chemically bonded to the foregoing FIBERSOL 2 or FIBERSOL 2H. The polymers are ones in which citric acid and glucose polymers are bonded through ester bonds. The molar ratio of the citric acid to the glucose polymer bonded together in the foregoing dextrin preferably ranges from 2:1 to 1:1 while taking into consideration applications thereof as food. Further, the foregoing ester bonds are preferably mono-ester bonds while taking into consideration the formation of water-soluble salts with minerals.

The glucose polymers serving as raw materials for preparing the citric acid-bound glucose polymers used in the present invention are reduced hardly digestible dextrin. Glucose polymers are reduced starch hydrolyzates, hardly digestible starch hydrolyzates, hardly digestible dextrins or hydrogenated products thereof (reduced hardly digestible dextrins). In this respect, the use of such reduced starch hydrolyzates and reduced hardly digestible dextrins involves glucose polymers not significantly pigmented during the reaction thereof with citric acid and therefore, the resulting glucose polymers have high commercial value. On the other hand, the use of hardly digestible starch hydrolyzates or hydrogenated products thereof would not only permit the impartment of the mineral-absorption promoting effect to the resulting products, but also permit the applications thereof as dietary fibers or low-caloric food.

The glucose polymers used herein may have a wide variety of polymerization degrees depending on the intended characteristic properties of the glucose polymers, but the degree of polymerization thereof preferably ranges from 4 to 123, more preferably 4 to 18 and most preferably 6 to 15, while taking into consideration the fact that they should be mixed with citric acid and then dried and converted into powder. When using a glucose polymer having a degree of polymerization higher than the foregoing upper limit, it sometimes forms insoluble matters upon dissolution thereof in water and accordingly, the use thereof would often be limited. On the other hand, the use of a glucose polymer having a degree of polymerization lower than the foregoing lower limit is not preferred since it is difficult to obtain a powdery product.

When using starch as raw materials for preparing such glucose polymers, the kinds thereof are not restricted to specific ones and usable herein as effective raw starch materials include, for instance, potato starch, sweet potato starch, corn starch, tapioca starch, and wheat starch.

Now, the method for the preparation of the citric acid-bound glucose polymer of the present invention will hereunder be described in more detail.

First, a glucose polymer and citric acid are admixed together and then they are dissolved in water to give an aqueous solution.

The mixing ratio of the glucose polymer to citric acid is appropriately selected depending on desired properties of the polymer to be formed, but it (molar ratio) ranges from 1:1 to 1:3 and preferably 1:2.5, in order to form desired polymeric products.

Incidentally, the number average molecular weight of hardly digestible dextrins, derivatives thereof, for instance, glucose polymers can be determined by fractionating them using a size exclusion HPLC column such as one comprising TSK Gel G6000PW$_{XL}$, G3000PW$_{XL}$ and G2500PW$_{XL}$ connected in series (they are all available from Tosoh Corporation) to thus separate the polymers on the basis of the molecular weights thereof and determining the molecular weights of the polymers in the light of the calibration curve prepared using pullulan as a reference material.

The amounts of the glucose polymer and citric acid to be dissolved in water are not limited to specific ranges and the amounts thereof may be as high as they can be dissolved in water, but the total amount of the glucose polymer and citric acid to be dissolved in water preferably ranges from 20 to 50 parts by mass and more preferably 30 to 40 parts by mass per 100 parts by mass of water. These components are in general dissolved in water under ordinary pressure at a temperature ranging from 10 to 60°C and currently at ordinary temperature while stirring the mixture at need.

The resulting aqueous solution is dried at a temperature preferably ranging from 95 to 110°C for 1 to 10 hours to thus give uniform powder and, in general, uniform amorphous powder. As a method for drying and converting the mixed aqueous solution of the glucose polymer and citric acid into a uniform powdery product, usable herein include, for instance, the spray drying technique, the drum-drying technique, and the freeze-drying technique, either of which may be efficiently be used in the present invention.

Then the resulting powdery product in its powdery state is subjected to an additional heat-treatment at a temperature preferably ranging from 100 to 160°C for a time period usually ranging from 1 to 20 hours, preferably 2 to 15 hours and more preferably 2 to 10 hours to thus obtain a desired citric acid-bound glucose polymer. Heat-treating devices usable in such a treatment may be a variety of devices currently used. Effectively used herein as such heat-treating devices include, for instance, those which permit continuous heat-treatments such as an oil bath and a rotary kiln; or a vacuum roasting device, an extruder, a drum dryer and a fluidized bed heating device.

The temperature of the powdery product during the heat-treatment preferably ranges from 100 to 160°C and more preferably 100 to 125°C. In this regard, the higher the reaction temperature, the higher the reaction rate. More specifically, if the heat-treatment is carried out at a temperature of higher than 125°C, the reaction can proceed at a high reaction rate, but water-insoluble matters are often formed during the reaction. However, any water-insoluble matter cannot be formed through the reaction carried out at a temperature ranging from 100 to 125°C.

In the reaction product thus obtained, the glucose polymer is principally bonded to citric acid through mono-ester bonds, while they bind quite rarely together through diester bonds.

The product obtained through the foregoing heat-treatment is not necessarily purified depending on the applications thereof, but the product can efficiently be purified while making use of the methods and devices used in the purification of the usual saccharides, for instance, a filtering device, a desalting device using an ion-exchange resin or a membrane separator, in particular, when the powdery product is used as, for instance, a food and feed.

The amount of the citric acid moieties bonded to the glucose polymer in the citric acid-bound glucose polymer thus purified can quantitatively and indirectly be determined by the determination of the rise and fall of the amount of free citric acid present in the composition observed before and after the reaction while making use of the HPLC technique. In addition, the type of ester bonds present in the citric acid-bound glucose polymer can be estimated or predicted by determining the amount of carboxyl groups present in the polymer according to the neutralization-titration technique.

The citric acid-bound glucose polymer thus prepared or the foregoing hardly digestible dextrin may be used alone or in any combination of at least two of them as a mineral-absorption promoter in the form of, for instance, a tablet, a granule, or a capsule. Moreover, the citric acid-bound glucose polymer or the hardly digestible dextrin may likewise be used by the incorporation thereof into a variety of beverages such as refreshing drinks, fermented beverages, and milk beverages; a variety of foods such as cereals, breads, confectionary, snuck food, and candies; feed or feeds such as those for domestic animals, poultry, and various kinds of pet animals. Further, the citric acid-bound glucose polymer or the hardly digestible dextrin may be used as an ingredient to be incorporated into, for instance, supplements for replenishing minerals or enteral nutrient compositions such as liquid diets.

When ingesting a food free of any mineral, the mineral-absorption promoter according to the present invention can be taken together with any mineral to thus allow the acceleration of the mineral-absorption. In this respect, when ingesting the mineral-absorption promoter according to the present invention in the form of a mineral salt, however, the salt may be ingested alone.

It would in general be sufficient that the mineral-absorption promoter of the present invention, which comprises, as an effective component, the hardly digestible dextrin or a derivative thereof is administered through the oral route in a daily dose generally ranging from 0.1 to 50 g and preferably 0.5 to 10 g for adult while subdividing the daily dose into 1 to 3 portions. The amount of these substances to be ingested may appropriately be controlled while taking into consideration various factors such as body weight and age of each particular subject.

Moreover, when adding the mineral-absorption promoter of the present invention to, for instance, a food or feed, it is in general sufficient to add the same to each intended substance in an amount preferably ranging from 1 to 20% by mass.

Then, the present invention will further be described in more detail below while taking, by way of example, a specific case in which the citric acid-bound glucose polymer is used as a calcium-absorption promoter.

The carboxyl groups of the citric acid-bound glucose polymer used as a calcium-absorption promoter are present in the polymer in the form of a salt with an alkali metal. For instance, when it is added to an acidic beverage, the glucose polymer in the form of its free state may be used without any additional treatment, but when using the same in a beverage or a food having an approximately neutral pH value, the glucose polymer is used in the form of an alkali metal salt, while taking note of the problems of taste and palatability thereof. Specific examples of such alkali metal or alkaline earth metal salts include potassium, sodium, calcium and magnesium salts.

The foregoing calcium-absorption promoter is preferably ingested in such a manner that the molar ratio of the calcium to be taken to the citric acid moieties present in the citric acid-bound glucose polymer ranges from 1:0.1 to 1:2, preferably 1:0.5 to 1:1.5, and more preferably 1:1. For instance, the amount of calcium to be ingested by Japanese adults ranges from 12.5 to 15 mM/day on the average and therefore, the amount of the citric acid-bound glucose polymer to be ingested in this case preferably ranges from 1.25 to 30 mM/day as expressed in terms of the amount of citric acid.

In the present invention, the currently used test for estimating mineral-incomings and mineral-outgoings can be employed for evaluating the ability thereof to absorb minerals observed for the mineral-absorption promoter of the present invention. For instance, rats are used as test animals and they are kept for 1 to 2 weeks while allowing them to freely take a mineral-containing test feed. In this connection, the amount of the feed ingested and the content of the minerals present in the feces collected for several days prior to the end of the test keeping period to thus calculate the apparent rate of mineral-absorption on the basis of the following equation 1. In addition, the apparent rate of mineral-absorption prior to the initiation of the test ($0^{th}$ week) can simultaneously be determined to thus calculate the rate of variation according to the following equation 2.

**Equation 1:**

$$\text{Apparent Rate of Mineral-Absorption (\%)} = 100 \times [(\text{ingested amount of minerals originated from feed}) - (\text{amount of minerals excreted in feces})]/(\text{ingested amount of minerals originated from feed});$$

**Equation 2:**

$$\text{Rate of Variation (\%)} = 100 \times [(\text{apparent rate of mineral-absorption}) - (\text{apparent rate of mineral-absorption observed at } 0^{th} \text{ week})]/(\text{apparent rate of mineral-absorption observed at } 0^{th} \text{ week})$$

Next, the mineral-solubilizing ability of the citric acid-bound glucose polymer was examined in vitro and the results thus obtained are given below as Reference Examples.

Reference Example: Test for Evaluating Ability of Candidate Substance to Promote Mineral-Solubilization in Phosphate-Buffered Saline

To 50 mL of phosphate-buffered saline whose final concentration was 16 mM, there was added 10 to 500 mg of sodium salt of reduced hardly digestible dextrin-citric acid ester prepared according to the method detailed later in the following Example 1 or reduced hardly digestible dextrin (FIBERSOL 2H (FS2H) available from Matsutani Chemical Industry Co., Ltd.: the hydrogenated product of hardly digestible dextrin having a number average molecular weight of about 2,000 and having a highly branched structure) (hereunder, both of them will collectively be referred to as "glucide") and then each mineral component was added thereto so that the concentration thereof was set at a level of 4 mM. In this respect, calcium chloride was used as a calcium-containing agent; magnesium chloride as a magnesium-containing agent; ferric chloride as an iron-containing agent; and zinc chloride as a zinc-containing agent, respectively. There were likewise prepared a sample free of any glucide and a sample free of any mineral component as positive and negative controls.

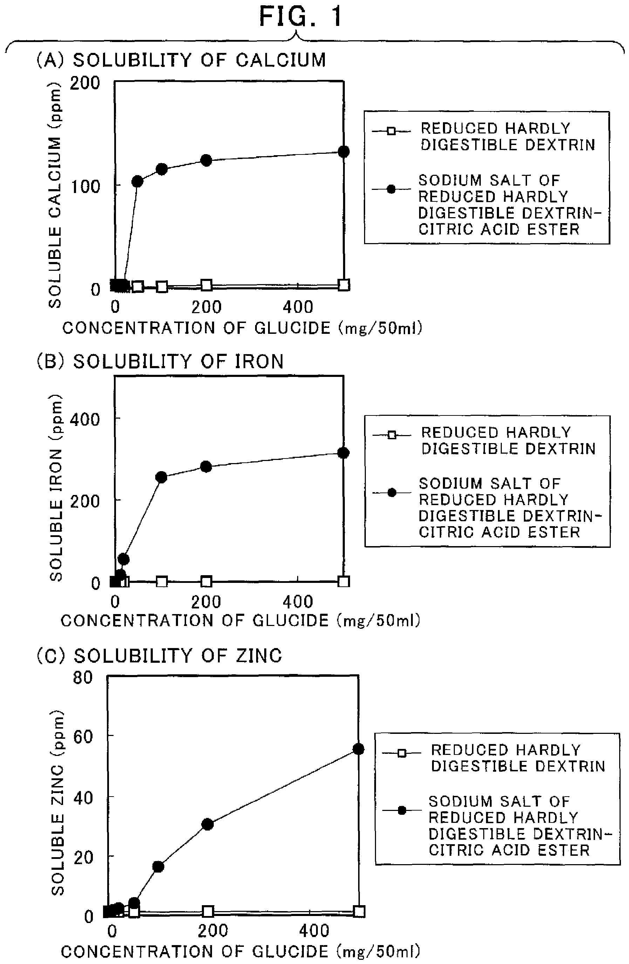

After maintaining each sample at 37 °C for one hour, the resulting supernatant was collected and the concentration of the minerals dissolved therein was determined according to the atomic absorption spectrometry. The results thus obtained are plotted on the attached Fig. 1. In this regard, magnesium chloride was not insolubilized at all even if the sample was free of any glucide and accordingly, the data concerning such samples are omitted from those plotted on Fig. 1.

The results plotted on Fig. 1 definitely indicate that the sodium salt of reduced hardly digestible dextrin-citric acid ester could significantly improve the solubility of minerals in the phosphate-buffered saline. On the other hand, there was not observed any such effect, at all, for the reduced hardly digestible dextrin. This result clearly indicates that the citric acid combined with the hardly digestible dextrin contributes to the improvement of the solubility of minerals.

The present invention will now be described in more specifically with reference to the following Examples, but the present invention is not restricted to these specific Examples at all.

Example 1: Preparation of Citric Acid-Bound Glucose Polymer

There was dissolved, in 23 kg of water, 8.1 kg (4.05 moles) of a reduced hardly digestible dextrin (FIBERSOL 2H (FS2H) available from Matsutani Chemical Industry Co., Ltd.: the hydrogenated product of hardly digestible dextrin having a number average molecular weight of about 2,000 and having a highly branched structure), with stirring; and then 1.9 kg (9.90 moles) of citric acid (available from U.S. Archer Daniels Midland Company) was blended with and dissolved in the foregoing aqueous solution. Subsequently, the resulting aqueous solution was dried in a spray dryer to thus give uniform powder of dextrin/citric acid mixture. Then 7 kg of the powder was subjected to a heat-treatment for 400 minutes while maintaining the temperature of the powder at 120 °C. In addition, the heat-treated powder was dissolved in water (to a concentration of 10% (w/w)) and the unreacted citric acid was removed using a loose reverse osmosis membrane (NTR-7470 available from Nitto Denko Corporation). The resulting dialyzate solution was dried in a spray dryer to convert it into powder and to thus give 5.5 kg of purified reduced hardly digestible dextrin-citric acid ester. In the reduced hardly digestible dextrin-citric acid ester thus purified, it was found that the reduced hardly digestible dextrin and citric acid bound one another in a molar ratio of 1:1.2 and that they bound together through mono-ester bonds. Moreover, this reduced hardly digestible dextrin-citric acid ester was dissolved in water (to a concentration of 30% (w/w)), the resulting aqueous solution was neutralized with sodium hydroxide, the neutralized solution was again dried in a spray dryer to form powder thereof in the form of a sodium salt of the reduced hardly digestible dextrin-citric acid ester.

Example 2: Test for Evaluating Mineral-Incomings and Mineral-Outgoings in Rats through Intake of Standard Calcium-Containing Feed

Five-week-old SD type male rats (24 animals) were preliminarily raised with a standard calcium-containing feed (calcium content of 0.5%) for one week, these animals were divided into a control group; a hardly digestible dextrin (FS2)-administered group; a reduced hardly digestible dextrin (FS2H)-administered group; and a group to which the sodium salt of the reduced hardly digestible dextrin-citric acid ester (FS2H/C. Na) prepared in Example 1 was administered, depending on the kinds of the feed fed thereto. Each feed was given, in two stages comprising a low dose (15 g/kg) stage and a high dose (30 g/kg) stage, to the animals of each corresponding group and the animals in each group (containing 8 animals each) were experimentally raised for one week. In the experimental raising, the animals were kept while allowing them to freely ingest the feed as specified in the following Table 1 and deionized water, under the dark and brightness cycle conditions at intervals of 12 hours. The feces of the animals of each test group were collected at the end of the preliminary keeping (0th week) and for the three days prior to the end of the first week, which were defined to be the terms for testing the mineral-incomings and mineral-outgoings in the test animals and the contents of calcium, magnesium, iron and zinc present in the feces were determined according to the atomic absorption spectrometry. The mineral intakes during the testing terms were calculated from the mineral content of the feed and the amount thereof ingested and the apparent rates of mineral-absorption were derived according to the foregoing equation 1.

Table 1: Standard Calcium-Containing Test Feeds (g/kg)

|  | Control | FS2[3)] | FS2H[4)] | FS2H/C.Na[5)] |
|---|---|---|---|---|
| Casein | 200 | 200 | 200 | 200 |
| Corn starch | 529.5 | 529.5 | 499.5 | 499.5 |
| Sucrose | 100 | 100 | 100 | 100 |
| Vitamin Mix[1)] | 10 | 10 | 10 | 10 |
| Mineral Mix[2)] | 35 | 35 | 35 | 35 |
| L-Cystine | 3 | 3 | 3 | 3 |
| Choline bitartrate | 2.5 | 2.5 | 2.5 | 2.5 |
| Soybean oil | 70 | 70 | 70 | 70 |
| Cellulose | 50 | 50 | 50 | 50 |
| t-Butyl hydroquinone | 0.014 | 0.014 | 0.014 | 0.014 |
| FS2[3)] | -- | 15 or 30 | -- | -- |
| FS2H[4)] | -- | -- | 15 or 30 | -- |
| FS2H/C.Na[5)] | -- | -- | -- | 15 or 30 |

(continued)

|  | Control | FS2[3] | FS2H[4] | FS2H/C.Na[5] |
|---|---|---|---|---|
| Calcium Content (%) | 0.5 | 0.5 | 0.5 | 0.5 |

1) AIN-93 Vitamin Mix (the vitamin mixture in the standard feed for mice and rats as established by the U.S. National Nutrients Research Laboratory; available from CLEA Japan, Inc.);
2) AIN-93G Mineral Mix (the mineral mixture in the standard feed for mice and rats in their breeding phase as established by the U.S. National Nutrients Research Laboratory; available from CLEA Japan, Inc.);
3) Hardly digestible dextrin;
4) Reduced hardly digestible dextrin;
5) Sodium salt of reduced hardly digestible dextrin-citric acid ester.

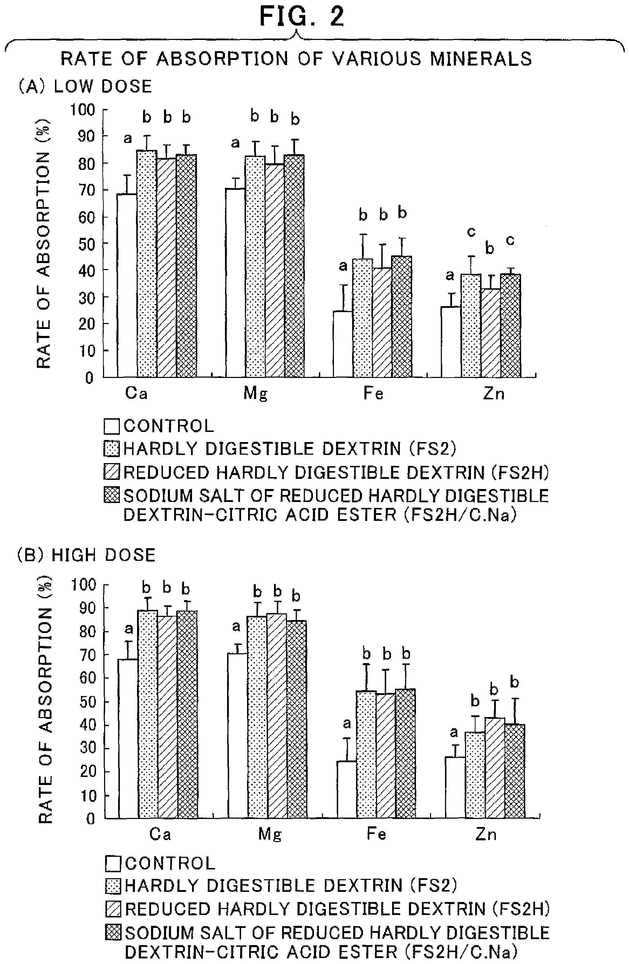

As shown in Fig. 2, the hardly digestible dextrin (FS2)-administered group, the reduced hardly digestible dextrin (FS2H)-administered group and the sodium salt of reduced hardly digestible dextrin-citric acid ester (FS2H/C. Na)-administered group could all significantly increase the rates of mineral-absorption in the both low dose (A) and high dose (B) stages, as compared with those observed for the control group.

Example 3: Test for Evaluating Calcium-Incomings and Calcium-Outgoings in Rats through Intake of Standard Calcium-Containing Feed

Nine-week-old SD type male rats (18 animals) were preliminarily raised with a standard calcium-containing feed (calcium content of 0.5%) for one week, these animals were divided into a control group; a reduced hardly digestible dextrin (FS2H)-administered group; and a group to which the sodium salt of the reduced hardly digestible dextrin-citric acid ester (FS2H/C. Na) prepared in Example 1 was administered, depending on the kinds of the feed fed thereto. The animals of each group (6 animals per group) were experimentally raised for 2 weeks. In the experimental raising, the animals of each group were allowed to freely ingest the corresponding feed as specified in the following Table 2 and deionized water, under the dark and brightness cycle conditions at intervals of 12 hours. The feces of the animals of each test group were collected at the end of the preliminary keeping (0th week), for the three days prior to the end of the first week and for the three days prior to the end of the second week, which were defined to be the terms for testing the mineral-incomings and mineral-outgoings in the test animals and the contents of calcium present in the feces thus collected were determined according to the Calcium C-Test Wako (WAKO Pure Chemical Industry Co., Ltd.). The calcium intakes during the testing terms were calculated from the calcium content of the feed and the amount thereof ingested and the apparent rates of mineral-absorption were derived according to the foregoing equation 1. Moreover, the rates of variation observed for the rates of calcium-absorption were likewise calculated according to the foregoing equation 2.

Table 2: Standard Calcium-Containing Test Feeds (g/kg)

|  | Control | FS2H[3] | FS2H/C.Na[4] |
|---|---|---|---|
| Casein | 200 | 200 | 200 |
| Corn starch | 529.5 | 499.5 | 499.5 |
| Sucrose | 100 | 100 | 100 |
| Vitamin NEx[1] | 10 | 10 | 10 |
| Mineral Mix[2] | 35 | 35 | 35 |
| L-Cystine | 3 | 3 | 3 |
| Choline bitartrate | 2.5 | 2.5 | 2.5 |
| Soybean oil | 70 | 70 | 70 |
| Cellulose | 50 | 50 | 50 |
| t-Butyl hydroquinone | 0.014 | 0.014 | 0.014 |
| FS2H[3] | -- | 30 | -- |
| FS2H/C.Na[4] | -- | -- | 30 |

(continued)

|  | Control | FS2H[3] | FS2H/C.Na[4] |
|---|---|---|---|
| Calcium Content (%) | 0.5 | 0.5 | 0.5 |

| 1) AIN-93 Vitamin Mix<br>2) AIN-93G Mineral Mix;<br>3) Reduced hardly digestible dextrin;<br>4) Sodium salt of reduced hardly digestible dextrin-citric acid ester. |
|---|

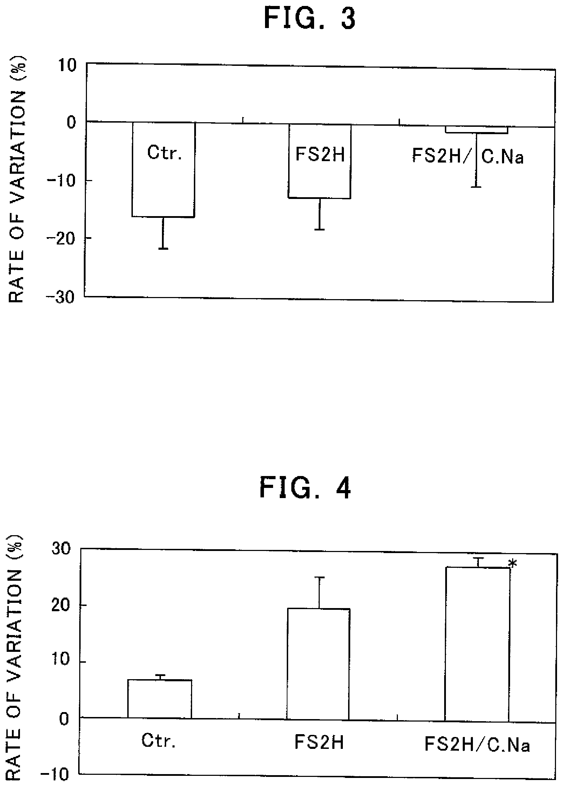

As shown in Fig. 3, the sodium salt of reduced hardly digestible dextrin-citric acid ester-administered group and the reduced hardly digestible dextrin-administered group could all significantly inhibit any reduction in the rates of mineral-absorption, as compared with that observed for the control group.

Example 4: Test for Evaluating Calcium-Incomings and Calcium-Outgoings in Rats through Intake of Feed Having Low Calcium Content

Twelve-week-old SD type male rats (12 animals) were preliminarily raised with a feed having a low calcium content (having a calcium content of 0.15%) for one week, these animals were divided into a control group; a reduced hardly digestible dextrin (FS2H)-administered group; and a sodium salt of reduced hardly digestible dextrin-citric acid ester (FS2H/C. Na)-administered group, depending on the kinds of the feed fed thereto. The animals of each group (4 animals per group) were experimentally raised for 2 weeks. Regarding each feed, to a feed having a low calcium content as a principal ingredient, there was added reduced hardly digestible dextrin or sodium salt of reduced hardly digestible dextrin-citric acid ester as an additional component. In the experimental raising, the animals of each group were allowed to freely ingest the corresponding feed as specified in the following Table 3 and deionized water. The apparent rates of mineral-absorption and the rates of variation observed for the rates of calcium-absorption were determined by repeating the same procedures used in Example 3.

Table 3: Test Feeds Having Low Calcium Content (g/kg)

|  | Control | FS2H | FS2H/C.Na |
|---|---|---|---|
| Casein | 200 | 200 | 200 |
| Corn starch | 529.5 | 499.5 | 499.5 |
| Sucrose | 96.25 | 96.25 | 96.25 |
| Vitamin Mix | 10 | 10 | 10 |
| Mineral Mix Lacking in Calcium[1] | 35 | 35 | 35 |
| L-Cystine | 3 | 3 | 3 |
| Choline bitartrate | 2.5 | 2.5 | 2.5 |
| Soybean oil | 70 | 70 | 70 |
| Cellulose | 50 | 50 | 50 |
| t-Butyl hydroquinone | 0.014 | 0.014 | 0.014 |
| FS2H | -- | 30 | -- |
| FS2H/C.Na | -- | -- | 30 |
| Calcium carbonate | 3.75 | 3.75 | 3.75 |
| Calcium Content (%) | 0.15 | 0.15 | 0.15 |
| 1) The product obtained by removing calcium from AIN-93G. | | | |

As shown in Fig. 4, the sodium salt of reduced hardly digestible dextrin-citric acid ester-administered group could significantly increase the rate of calcium- absorption as compared with that observed for the control group. Moreover, the reduced hardly digestible dextrin-administered group could likewise increase the rate of calcium-absorption, but the reduction thereof was not considered to be significant as compared with that observed for the control group. The effect of the administration of the sodium salt of reduced hardly digestible dextrin-citric acid ester on the improvement of the rate of calcium-absorption observed when the animals ingested a feed lacking in calcium was proved to be conspicuous as compared with that observed when the animals ingested a standard calcium-containing feed (see Example 3). This fact clearly suggests that the rate of calcium-absorption would be increased due to an increase in the molar ratio of the citric acid-bound glucose polymer to the amount of calcium upon the intake thereof. In fact, the foregoing molar ratio used in Example 3 was found to be 1:0.13, while that used in Example 4 was 1:0.44.

**Example 5:** Preparation of Tablet

Tablets of the calcium-absorption promoter of the present invention were prepared according to the formulation specified in the following Table 4:

Table 4:

| Raw Material | Amt. Blended (w/w %) |
|---|---|
| Sodium salt of reduced hardly digestible dextrin-citric acid ester (FS2H/C.Na) | 40 |
| Fine Particles for Direct Compressing No. 209 (Fuji Chemical Industry Co., Ltd.)[1] | 48 |
| Crystalline cellulose | 10 |
| Magnesium stearate | 2 |
| 1) A mixture comprising magnesium meta-silicate aluminate (20%), corn starch (30%) and lactose (50%). | |

The foregoing raw materials were uniformly blended together and then the resulting mixed powder was compressed to give tablets (200 mg each).

**Reference Example 6**: Preparation of Calcium-Enriched Beverage

Citric acid-bound reduced hardly digestible dextrin was neutralized with calcium carbonate according to the method similar to that used in Example 1 to give a calcium salt thereof (FS2H/C.Ca), FS2H/C.Ca was found to be soluble in water and accordingly, it easily permitted the preparation of a transparent aqueous solution having a concentration of at least 50% (w/v). Then a calcium-enriched beverage was prepared using FS2H/C.Ca according to the formulation specified in the following Table 5:

Table 5

| Raw Material | Amt. Blended (w/v %) |
|---|---|
| FS2H/C.Ca | 3.92 |
| Granulated sugar | 7.00 |
| Citric acid | 0.35 |
| Vitamin mixture | 0.20 |
| Common salt | 0.005 |
| Calcium chloride | 0.008 |
| Flavor | 0.10 |
| water | added to 100ml |

Example 7: Preparation of Dog Food

A dog food was prepared according to the formulation detailed in the following Table 6:

Table 6

| Raw Material | Amt. Blended (w/w %) |
|---|---|
| FS2H/C.Na | 5.3 |

(continued)

| Raw Material | Amt. Blended (w/w %) |
|---|---|
| Corn | 30.0 |
| Wheat flour | 33.0 |
| Soybean meal | 21.0 |
| Defatted rice bran | 5.5 |
| Meat meal | 5.0 |
| Mineral mixture | 0.2 |

**Reference Example 8**: Preparation of Tablets

Tablets of the mineral-absorption promoter of the present invention were prepared according to the formulation specified in the following Table 7:

Table 7:

| Raw Material | Amt. Blended (w/w %) |
|---|---|
| Hardly digestible dextrin (FS2 available from Matsutani Chemical Industry Co., Ltd.) | 40 |
| Fine Particles for Direct Compressing No. 209 (Fuji Chemical Industry Co., Ltd.)[1] | 48 |
| Crystalline cellulose | 10 |
| Magnesium stearate | 2 |
| 1) A mixture comprising magnesium meta-silicate aluminate (20%), corn starch (30%) and lactose (50%). | |

The foregoing raw materials were uniformly blended together and then the resulting mixed powder was compressed to give tablets (200 mg each).

**Reference Example 9**: Preparation of Magnesium-Enriched Beverage

Citric acid-bound reduced hardly digestible dextrin was neutralized with magnesium carbonate according to the method similar to that used in Example 1 to give a magnesium salt thereof (FS2H/C.Mg). FS2H/C.Mg was found to be soluble in water and accordingly, it easily permitted the preparation of a transparent aqueous solution having a concentration of at least 50% (w/v). Then a magnesium-enriched beverage was prepared using FS2H/C.Mg according to the formulation specified in the following Table 8:

Table 8

| Raw Material | Amt. Blended (w/v %) |
|---|---|
| FS2H/C.Mg | 2.00 |
| Granulated sugar | 7.00 |
| Citric acid | 0.35 |
| Vitamin mixture | 0.20 |
| Common salt | 0.005 |
| Potassium chloride | 0.008 |
| Flavor | 0.10 |
| Water | added to 100ml |

**Reference Example 10**: Preparation of Dog Food

A dog food was prepared according to the formulation detailed in the following Table 9:

Table 9

| Raw Material | Amt. Blended (w/w %) |
|---|---|
| Reduced hardly digestible dextrin (FS2H available from Matsutani Chemical Industry Co., Ltd.) | 5.3 |
| Corn | 30.0 |
| Wheat flour | 33.0 |
| Soybean meal | 21.0 |
| Defatted rice bran | 5.5 |
| Meat meal | 5.0 |
| Mineral mixture | 0.2 |

**Reference Example 11**: Preparation of Supplement

A mixture for calcium-supplement was prepared according to the formulation specified in the following Table 10, the resulting mixture was blended with water, the mixture was granulated and then dried.

Table 10

| Raw Material | Amt. Blended (w/w %) |
|---|---|
| Egg-shell calcium | 55.0 |
| Corn starch | 30.8 |
| Crystalline cellulose | 2.5 |
| CMC calcium | 1.7 |
| Hardly digestible dextrin (FS2 available from Matsutani Chemical Industry Co., Ltd.) | 10.0 |

The dried mixture was pulverized and then classified to thus form a powdery product for compressing. To the powdery product, there was added a sucrose fatty acid ester as a lubricant such that the content thereof was equal to 2% (w/w) and then the powdery mixture was compressed into tablets having an average weight of 0.35g.

Example 12: Preparation of Enteral Nutrient

An enteral nutrient was prepared according to the formulation specified in the following Table 11:

Table 11

| Raw Material | Amt. Blended (per 250 ml) |
|---|---|
| Sodium caseinate | 5.9 g |
| Sodium calcium caseinate | 2.7 g |
| Soybean protein | 1.3 g |
| White refined sugar | 9.8 g |
| Dextrin | 24.5 g |
| Corn oil | 8.3 g |
| Soybean phospholipid | 0.4 g |

(continued)

| Raw Material | Amt. Blended (per 250 ml) |
|---|---|
| Vitamin A | 625 IU |
| Vitamin D | 50 IU |
| Vitamin E (Tocopherol acetate) | 8.23 mg |
| Vitamin K | 17.51 $\mu$g |
| Vitamin B1 (Thiamine hydrochloride) | 0.38 mg |
| Vitamin B2 | 0.43 mg |
| Vitamin B6 (Pyridoxine hydrochloride) | 0.50 mg |
| Vitamin B12 (Cyanocobalamin) | 1.5 $\mu$g |
| Vitamin C | 38 mg |
| Niacin | 5.0 mg |
| Folic acid | 50 $\mu$g |
| Calcium pantothenate | 1.36 mg |
| Biotin | 38 $\mu$g |
| Choline chloride | 0.15 g |
| Calcium phosphate | 0.3 g |
| Magnesium chloride | 0.41 g |
| Potassium citrate | 0.46 g |
| Potassium chloride | 0.30 g |
| Sodium citrate | 0.39 g |
| Zinc sulfate | 16.49 mg |
| Iron sulfate | 11.20 mg |
| Manganese chloride | 1.80 mg |
| Copper sulfate | 0.98 mg |
| Potassium hydroxide | 24 mg |
| Citric acid | 25 mg |
| Sodium hydrogen carbonate | 76.5 $\mu$g |
| Reduced hardly digestible dextrin (FS2H available from Matsutani Chemical Industry Co., Ltd.) | 7.5 g |

Brief Description of Drawings

Fig. 1 is a graph showing the results obtained in the test for promoting mineral-dissolution in phosphate-buffered saline carried out in Reference Example.
Fig. 2 is a graph illustrating the effect of hardly digestible dextrins on the rate of mineral-absorption in rats which ingest a standard calcium-containing feed, observed in Example 2.
Fig. 3 illustrates the rate of variation in the rate of calcium-absorption observed when a standard calcium-containing feed was fed to test animals. In this figure, Ctr. represents a control group; FS2H a reduced hardly digestible dextrin-administered group; and FS2H/C.Na a sodium salt of reduced hardly digestible dextrin-citric acid ester-administered group, respectively.

Fig. 4 illustrates the rate of variation in the rate of calcium-absorption observed when a feed lacking in calcium was fed to test animals. In this figure, the symbols used are the same as those used in Fig. 3.

## Claims

1. A mineral-absorption promoter comprising an alkali metal salt of a citric acid-bound reduced hardly digestible dextrin as an effective component, wherein the ratio of the reduced hardly digestible dextrin to citric acid ranges from 1:1 to 1:3.

2. The mineral-absorption promoter as set forth in claim 1, wherein the reduced hardly digestible dextrin is water-soluble dietary fibers obtained by treating a hydrogenated product of roasted dextrin with $\alpha$-amylase and glucoamylase.

3. The mineral-absorption promoter as set forth in claim 1 or 2 for use in preventing insufficient or deficient mineral-absorption by the intestinal tracts.

4. The mineral-absorption promoter for use in preventing insufficient or deficient mineral-absorption as set forth in claim 3, wherein the mineral is at least one member selected from the group consisting of calcium, magnesium, iron and zinc.

5. The mineral-absorption promoter for use in preventing insufficient or deficient mineral-absorption as set forth in claim 4, wherein the mineral is at least one member selected from the group consisting of magnesium, iron and zinc.

6. Food comprising a mineral-absorption promoter as set forth in any one of claims 1 to 5.

7. Feed comprising a mineral-absorption promoter as set forth in any one of claims 1 to 5.


## Patentansprüche

1. Mineralabsorptionsförderer, der ein Alkalimetallsalz eines Zitronensäuregebundenen reduzierten schwerverdaulichen Dextrins als wirksamen Bestandteil umfasst, wobei das Verhältnis des reduzierten schwerverdaulichen Dextrins zu Zitronensäure im Bereich von 1:1 bis 1:3 liegt.

2. Mineralabsorptionsförderer nach Anspruch 1, wobei das reduzierte schwerverdauliche Dextrin wasserlösliche Ballaststoffe darstellt, die durch Behandeln eines hydrierten Produkts aus geröstetem Dextrin mit $\alpha$-Amylase und Glucoamylase erhalten werden.

3. Mineralabsorptionsförderer nach Anspruch 1 oder 2 zur Verwendung bei der Vorbeugung einer unzureichenden oder mangelhaften Mineralabsorption durch den Darmtrakt.

4. Mineralabsorptionsförderer zur Verwendung bei der Vorbeugung einer unzureichenden oder mangelhaften Mineralabsorption nach Anspruch 3, wobei das Mineral mindestens ein Mitglied ausgewählt aus der Gruppe, bestehend aus Calcium, Magnesium, Eisen und Zink ist.

5. Mineralabsorptionsförderer zur Verwendung bei der Vorbeugung einer unzureichenden oder mangelhaften Mineralabsorption nach Anspruch 4, wobei das Mineral mindestens ein Mitglied ausgewählt aus der Gruppe, bestehend aus Magnesium, Eisen und Zink ist.

6. Nahrungsmittel, das einen Mineralabsorptionsförderer nach einem der Ansprüche 1 bis 5 umfasst.

7. Futtermittel, das einen Mineralabsorptionsförderer nach einem der Ansprüche 1 bis 5 umfasst.


## Revendications

1. Promoteur d'absorption de minéral comprenant un sel de métal alcalin d'une dextrine difficilement digestible réduite liée à l'acide citrique comme un constituant efficace, où le rapport de la dextrine difficilement digestible réduite à l'acide citrique est de 1:1 à 1:3.

2. Promoteur d'absorption de minéral selon la revendication 1, où la dextrine difficilement digestible réduite est des fibres alimentaires solubles dans l'eau obtenues par traitement d'un produit hydrogéné de dextrine grillée avec de l'a-amylase et du glucoamylase.

3. Promoteur d'absorption de minéral selon la revendication 1 ou 2 pour une utilisation dans la prévention d'une absorption de minéral insuffisante ou déficiente par les voies intestinales.

4. Promoteur d'absorption de minéral pour une utilisation dans la prévention d'une absorption de minéral insuffisante ou déficiente selon la revendication 3, où le minéral est au moins un élément choisi dans le groupe constitué de calcium, magnésium, fer et zinc.

5. Promoteur d'absorption de minéral pour une utilisation dans la prévention d'une absorption de minéral insuffisante ou déficiente selon la revendication 4, où le minéral est au moins un élément choisi dans le groupe constitué de magnésium, fer et zinc.

6. Aliment comprenant un promoteur d'absorption de minéral selon l'une quelconque des revendications 1 à 5.

7. Aliment comprenant un promoteur d'absorption de minéral selon l'une quelconque des revendications 1 à 5.

# FIG. 1

(A) SOLUBILITY OF CALCIUM

CONCENTRATION OF GLUCIDE (mg/50ml)

Legend:
- □ REDUCED HARDLY DIGESTIBLE DEXTRIN
- ● SODIUM SALT OF REDUCED HARDLY DIGESTIBLE DEXTRIN-CITRIC ACID ESTER

(B) SOLUBILITY OF IRON

CONCENTRATION OF GLUCIDE (mg/50ml)

Legend:
- □ REDUCED HARDLY DIGESTIBLE DEXTRIN
- ● SODIUM SALT OF REDUCED HARDLY DIGESTIBLE DEXTRIN-CITRIC ACID ESTER

(C) SOLUBILITY OF ZINC

CONCENTRATION OF GLUCIDE (mg/50ml)

Legend:
- □ REDUCED HARDLY DIGESTIBLE DEXTRIN
- ● SODIUM SALT OF REDUCED HARDLY DIGESTIBLE DEXTRIN-CITRIC ACID ESTER

# FIG. 2

RATE OF ABSORPTION OF VARIOUS MINERALS

(A) LOW DOSE

☐ CONTROL
▨ HARDLY DIGESTIBLE DEXTRIN (FS2)
▨ REDUCED HARDLY DIGESTIBLE DEXTRIN (FS2H)
▨ SODIUM SALT OF REDUCED HARDLY DIGESTIBLE
DEXTRIN-CITRIC ACID ESTER (FS2H/C.Na)

(B) HIGH DOSE

☐ CONTROL
▨ HARDLY DIGESTIBLE DEXTRIN (FS2)
▨ REDUCED HARDLY DIGESTIBLE DEXTRIN (FS2H)
▨ SODIUM SALT OF REDUCED HARDLY DIGESTIBLE
DEXTRIN-CITRIC ACID ESTER (FS2H/C.Na)

## FIG. 3

## FIG. 4

**EP 1 964 855 B1**

**REFERENCES CITED IN THE DESCRIPTION**

**Patent documents cited in the description**

- JP 3240470 A
- JP 5284939 A
- JP 56097248 A
- JP 8104696 A
- JP 2000157186 A
- JP 7252156 A
- JP 7067575 A
- JP 4043624 B
- JP 20045243661 A
- JP 2004524849 A
- JP 2004307768 A

**Non-patent literature cited in the description**

- *New Food Industry,* 2001, vol. 43 (12), 35-44
- *Bulletin of the Dietary Fiber Society in Japan,* 2005, vol. 9 (1), 34-46
- *Journal of Nutrition,* 2000, vol. 130 (5), 1267-1273